Europäisches Patentamt

European Patent Office

Office européen des brevets

(19)

(11) **EP 0 649 540 B1**

(12) ## EUROPÄISCHE PATENTSCHRIFT

(45) Veröffentlichungstag und Bekanntmachung des Hinweises auf die Patenterteilung:
**22.01.1997  Patentblatt 1997/04**

(21) Anmeldenummer: **93915847.3**

(22) Anmeldetag: **08.07.1993**

(51) Int Cl.[6]: **G01T 1/163**, G01T 1/167

(86) Internationale Anmeldenummer:
**PCT/EP93/01783**

(87) Internationale Veröffentlichungsnummer:
**WO 94/01789 (20.01.1994 Gazette 1994/03)**

(54) **DETEKTORSYSTEM ZUR DIREKTEN INTERNEN DOSIMETRIE AN EINER PERSON**

DETECTOR SYSTEM FOR DIRECT INTERNAL DOSIMETRY IN HUMAN BEINGS

SYSTEME DETECTEUR DE DOSIMETRIE INTERNE DIRECTE POUR ETRES HUMAINS

(84) Benannte Vertragsstaaten:
**CH DE FR GB IT LI**

(30) Priorität: **10.07.1992  DE 4222661**

(43) Veröffentlichungstag der Anmeldung:
**26.04.1995  Patentblatt 1995/17**

(73) Patentinhaber: **Forschungszentrum Karlsruhe GmbH**
**76133 Karlsruhe (DE)**

(72) Erfinder: **DOERFEL, Hans**
**D-76227 Karlsruhe (DE)**

(74) Vertreter: **Rückert, Friedrich, Dr.**
**Forschungszentrum Karlsruhe GmbH**
**Stabsabteilung**
**Patente und Lizenzen**
**Weberstrasse 5**
**76133 Karlsruhe (DE)**

(56) Entgegenhaltungen:
**EP-A- 0 196 354**

- **KERNTECHNIK Bd. 19, Nr. 11, November 1977, MUNCHEN GERMANY Seite 507 CANBERRA ELEKTRONIK GMBH 'Gamma-Inkorporationsmessplatz'**
- **HEALTH PHYSICS Bd. 16, Nr. 6, Juni 1969, IRELAND Seiten 719 - 729 CHHABRA A S 'a whole-body counter for routine monitoring'**

**Beschreibung**

Die Erfindung betrifft ein Detektorsystem zur direkten internen Dosimetrie an einer Person nach dem Oberbegriff des Patentanspruchs 1, wie es aus IEEE Transactions on Nuclear Science, Vol. N5-J4, No.1, 1987, S.606-610 bekannt ist.

Aus Kerntechnik, Bd. 19, Nr. 11, November 1977, München, Germany, Seite 507; "Gamma-Inkorporationsmeßplatz" ist ebenfalls ein Detektorsystem der gattungsgemäßen Art bekannt.

Des weiteren ist aus der EP-A-0196 354 ein Detektorsystem bekannt, bei welchem mit zwei Detektoren der Schilddrüsenbereich und der Verdauungstrakt erfaßt werden, während der Patient auf einem Stuhl sitzt.

Bei der Inkorpationsüberwachung besteht die Aufgabe in der Bestimmung der Aktivitätszufuhr bei beruflich strahlenexponierten Personen bzw. in der Kontrolle darüber, daß die durch den Gesetzgeber vorgegebenen Grenzwerte der Aktivitätszufuhr nicht überschritten werden. Darüber hinaus muß durch die Überwachung auch sichergestellt werden, daß die aus der Aktivitätszufuhr resultierenden Folgedosen die vom Gesetzgeber vorgegebenen Grenzwerte der Körper- bzw. Organdosis nicht überschreiten.

Die Inkorporationsüberwachung kann durch Messung der Aktivität vor Eintritt in den Körper (Raumluft- bzw. Atemluftüberwachung), durch Messung der Aktivität nach Eintritt in den Körper (Direktmessung) oder durch Messung der Aktivität nach Austritt aus dem Körper (Ausscheidungsmessung) erfolgen. Im allgemeinen liefert die Direktmessung die zuverlässigsten Aussagen über die Aktivitätszufuhr und die resultierende Äquivalentdosis. Die Direktmessung ist allerdings nur bei solchen Radionukliden anwendbar, die eine Gamma- oder Röntgen-Strahlung mit hinreichender Energie und Intensität emittieren. Diese Voraussetzung ist in vielen Bereichen der Kerntechnik und der Medizin gegeben, so daß in diesen Bereichen die Direktmessung im allgemeinen bevorzugt wird.

Die Direktmessung der Körperaktivität erfolgt mit Ganz- oder Teilkörperzählern, die bei Meßzeiten zwischen 5 min und 20 min die Aktivität im Gesamtkörper bzw. in bestimmten Teilbereichen des Körpers erfassen. Die zu überwachenden Personen werden in regelmäßigen Abständen untersucht, wobei sich die Überwachungsfrequenz nach dem Grenzwert der Aktivitätszufuhr, der biologischen Halbwertszeit und der unteren Nachweisgrenze des Ganz- oder Teilkörperzählers richtet. Im allgemeinen liegen die überwachungsfrequenzen zwischen einer Messung und zwölf Messungen pro Jahr.

Wird bei einer dieser routinemäßigen Messungen eine signifikante Körperaktivität festgestellt, so muß zunächst die ursächliche Aktivitätszufuhr und im zweiten Schritt die resultierende Äquivalentdosis abgeschätzt werden. Hierfür werden die folgenden Informationen benötigt.

- Inkorporationszeitpunkt bzw. zeitliches Muster der Aktivitätszufuhr

- Zufuhrweg und chemische Form der zugeführten Aktivität

- Individuelles Stoffwechselverhalten

- Beitrag früherer Inkorporationen zum aktuellen Meßwert

Diese Informationen sind im allgemeinen nicht oder nur unzureichend verfügbar, so daß die Abschätzung mit erheblichen Fehlern behaftet sein kann. Bei Nukliden mit relativ langer biologischer Halbwertszeit und mit relativ einfachem und gut bekanntem Stoffwechselverhalten wie z.B. Cs-137 ist je nach Überwachungsfrequenz mit Fehlern zwischen etwa 25 % und einem Faktor 2 zu rechnen. Bei den übrigen Nukliden kann der Fehler durchaus auch über dem Faktor 2 liegen.

Wegen der Vielfalt der möglichen Inkorporationsmuster ist es außerordentlich schwer, allgemeingültige Verfahren zur Abschätzung der Aktivitätszufuhr und der resultierenden Äquivalentdosis zu definieren. Die in der Richtlinie zur Durchführung der Strahlenschutzverordnung definierten Verfahren stellen notwendigerweise einen Kompromiß zwischen der Übersichtlichkeit der Darstellung und der Vielfalt der erforderlichen Detailinformationen dar. Aus diesem Grund gehen bei der praktischen Umsetzung der Richtlinie im allgemeinen zahlreiche Unsicherheitsfaktoren ein. Diese Unsicherheitsfaktoren führen unter anderem zu den folgenden Schwierigkeiten:

- Es ist praktisch unmöglich, allgemeine Qualifikationsmerkmale wie Erkennungsgrenze, Nachweisgrenze und Vertrauensbereich für die Aktivitätszufuhr und die resultierende Äquivalentdosis anzugeben. Es ist daher sehr schwierig, verschiedene überwachungsverfahren miteinander zu vergleichen. Auch ist es sehr schwierig, Mindestanforderungen an Inkorporationsmeßstellen zu definieren.

- Es ist sehr schwierig, die Meßergebnisse so zu dokumentieren, daß sie bei einer späteren überprüfung noch nachvollziehbar sind. Diese Schwierigkeiten treten insbesondere bei der Dokumentation der Meßergebnisse in den Strahlenpässen von Personen mit wechselndem Arbeitsplatz auf. Die Schwierigkeiten werden in noch weit größerem Umfang auftreten, wenn die Ergebnisse der inneren Strahlenexposition im zentralen Dosisregister dokumentiert werden sollen.

-   Wird eine Person mit einer gegebenen Inkorporation in verschiedenen Institutionen untersucht, so werden erfahrungsgemäß häufig verschiedene Aktivitätszufuhren bzw. verschiedene Äquivalentdosen abgeschätzt. Dies kann insbesondere bei Personen mit wechselndem Arbeitsplatz zu großer Verwirrung führen.

Aufgabe der Erfindung ist es, ein Detektorsystem der eingangs genannten Art zur Verfügung zu stellen, mit dem die effektive Aquivalenzdosis eines Probanden direkt gemessen werden kann.

Diese Aufgabe wird durch die kennzeichnenden Merkmale des Patentanspruchs 1 gelöst. Die Unteransprüche beschreiben vorteilhafte Ausgestaltungen der Detektoranordnung.

Die mit herkömmlichen Ganzkörperzählern nachweisbaren Nuklide zeichnen sich im allgemeinen dadurch aus, daß der Zerfall dieser Nuklide von der Emission einer intensiven Gamma-Strahlung mit einer Energie von mehr als 100 keV begleitet wird. Befindet sich ein solches Nuklid in einem bestimmten Organ oder Gewebe des Körpers, so besteht ein definierter Zusammenhang zwischen der inkorporierten Aktivität und dem Photonenfluß an der Körperoberfläche. Auf diesem Zusammenhang basiert die herkömmliche Ganzkörpermessung.

Darüber hinaus besteht auch ein definierter Zusammenhang zwischen dem Photonenfluß an bestimmten Punkten der Körperoberfläche und der durch die inkorporierte Aktivität bedingten Äquivalentdosisleistung. Dieser Zusammenhang kann unter bestimmten Voraussetzungen zur direkten Messung der Äquivalentdosisleistung bei innerer Strahlenexposition herangezogen werden.

Das auf diesem Zusammenhang basierende Verfahren wird im folgenden als direkte interne Dosimetrie bezeichnet.

Nach dem dosimetrischen Konzept der Internationalen Strahlenschutzkomission gilt für die Äquivalentdosisleistung bei innerer Strahlenexposition die folgende Beziehung:

$$H'(T,S) = 1{,}38 \ 10^4 \cdot A_s \cdot SEE(T,S) \tag{1}$$

Dabei ist

$H'(T,S)$     die durch eine Nukliddeposition in einem bestimmten Quell-Organ S in einem bestimmten Target-Organ T erzeugte Äquivalentdosisleistung in nSv/d

$A_s$     die Aktivität der Nukliddeposition im Quell-Organ S in Bq

$SEE(T,S)$     die sogenannte spezifische effektive Energie von S bezogen auf T (die pro Zerfall des in S deponierten Nuklids in T pro Masseneinheit absorbierte Energie, multipliziert mit dem der Strahlenqualität des betreffenden Nuklids entsprechenden Qualitätsfaktor) in MeV/g/Zerfall; die SEE-Faktoren sind für alle wichtigen Nuklide in Limits for Intakes of Radionuclides by Workers, ICRP Publication 30, Part 1, Annals of the ICRP, Vol. 2, 3/4, Pergamon Press, Oxford 1979 tabelliert.

Der Beitrag des Target-Organs T zur effektiven Äquivalentdosisleistung ergibt sich aus Gl. (1) durch Multiplikation mit dem organspezifischen Wichtungsfaktor $w_T$:

$$H_{eff}'(T,S) = 1{,}38 \cdot 10^4 \cdot A_S \cdot W_T \cdot SEE(T,S) \tag{2}$$

Bei gammastrahlenden Nukliden sind im allgemeinen mehrere Target-Organe betroffen, so daß sich die gesamte effektive Äquivalentdosisleistung als Summe der Einzelbeträge aller Target-Organe ergibt:

$$H_{eff}'(S) = 1{,}38 \cdot 10^4 \cdot A_S \cdot \Sigma_T \, [W_T \cdot SEE(T,S)] = A_S \cdot h(S) \tag{3}$$

mit

$$h(S) = 1{,}38 \cdot 10^4 \cdot \Sigma_T \, [W_T \cdot SEE(T,S)] \tag{4}$$

Der auf diese Weise definierte effektive Äquivalentdosisleistungsfaktor h(S) gestattet die direkte Berechnung der effektiven Äquivalentdosisleistung aus der Aktivität im Quell-Organ S.

Ist die inkorporierte Aktivität auf mehrere Quell-Organe verteilt, so ergibt sich die gesamte effektive Äquivalentdosisleistung als Summe der Beiträge aller Quell-Organe:

$$H_{eff}' = \Sigma_S [H_{eff}'(S)] = \Sigma_S [A_S \cdot h(S)] \tag{5}$$

Tabelle 1:

| Depot S | Dosisleistungsfaktor h(S) (nSv/d pro Bq Co-60) |
|---------|------------------------------------------------|
| Ganzkörper | 0,20 |
| Lunge | 0,43 |
| Leber | 0,35 |
| Dünndarm | 0,72 |
| Oberer Dickdarm | 0,80 |
| Unterer Dickdarm | 1,20 |

Die Tabelle 1 zeigt als Beispiel einige Werte von h(S) für Co-60. Demnach führt eine zeitlich konstante - bzw. eine zeitlich gemittelte - homogene Ganzkörperaktivität in Höhe von 1 Bq Co60 zu einer effektiven Äquivalentdosisleistung in Höhe von 0,20 nSv/d. Befindet sich die Co-60-Aktivität in einem dosisrelevanten Organ, so ist die resultierende effektive Äquivalentdosisleistung entsprechend höher. So führt beispielsweise 1 Bq Co-60 in der Lunge zu 0,43 nSv/d und im unteren Dickdarm zu 1,2 nSv/d.

Zur Direktmessung der effektiven Äquivalentdosisleistung benötigt man ein Detektorsystem, dessen Systemempfindlichkeit in geeigneter Weise an die Faktoren h(S) angepaßt worden ist. Aus den Zahlenwerten h(S) ergibt sich unmittelbar, daß ein solches Detektorsystem aus mehreren Detektoren bestehen muß.

Bei der weiteren Betrachtung wird die Anzahl der Detektoren zunächst offengehalten. Die Zählrate des i-ten Detektors für eine Nukliddeposition im Quell-Organ S sei

$$Z_i(S) = e_i(S) \cdot A_S$$

Hierbei ist

$Z_i(S)$       die Zählrate des i-ten Detektors für Nukliddepositionen im Quellorgan S in imp/s
$e_i(s)$       die Empfindlichkeit des i-ten Detektors für Nukliddepositionen im Quell-Organ S in imp/s/Bq
$A_S$       die Aktivität im Quell-Organ S in Bq

Der Meßwert des Systems sei die folgende Linearkombination der Zählraten der einzelnen Detektoren:

$$M(S) = \Sigma_i[\alpha_i \cdot Z_i(S)] = \Sigma_i[\alpha_i \cdot e_i(S)] A_s = m(S) \cdot A_s \tag{7}$$

Hierbei ist

$M(S)$       der Meßwert des Systems für Nukliddepositionen im Quellorgan S in imp/s
$\alpha_i$       ein Wichtungsfaktor für den i-ten Detektor mit der Normierung $\Sigma_i \alpha_i = 1$
$A_s$       die Aktivität im Quell-Organ S in Bq
$m(S)$       die Systemempfindlichkeit für Nukliddepositionen im Quell-Organ S in imp/s/Bq

$$m(S) = \Sigma_i[\alpha_i \, e_i(S)]$$

Ist die inkorporierte Aktivität auf mehrere Quell-Organe verteilt, so gilt für den Meßwert:

4

$$M = \Sigma_s M(S) = \Sigma_s[m(S) \cdot A_s] \tag{8}$$

bzw.

$$M = \Sigma_i[\alpha_i \cdot \Sigma_s Z_i(S)] = \Sigma_i[\alpha_i \cdot Z_i] \tag{9}$$

wobei $Z_i$ die Gesamtimpulsrate des i-ten Detektors darstellt.

Wenn nun bei den nachzuweisenden Nukliden für alle möglicherweise betroffenen Quell-Organe die Beziehung

$$h(s) = K \cdot m(S) \tag{10}$$

besteht, wobei K ein konstanter Kalibrierfaktor ist, dann kann die effektive Äquivalentdosisleistung entsprechend der Beziehung

$$H_{eff}' = \Sigma_s[h(S) \cdot A_s] = \Sigma_s[K \cdot m(S) \cdot A_s] = K \cdot M \tag{11}$$

unmittelbar aus dem Meßwert M berechnet werden. Hierin besteht das Prinzip der direkten Messung der effektiven Äquivalentdosisleistung bei innerer Strahlenexposition.

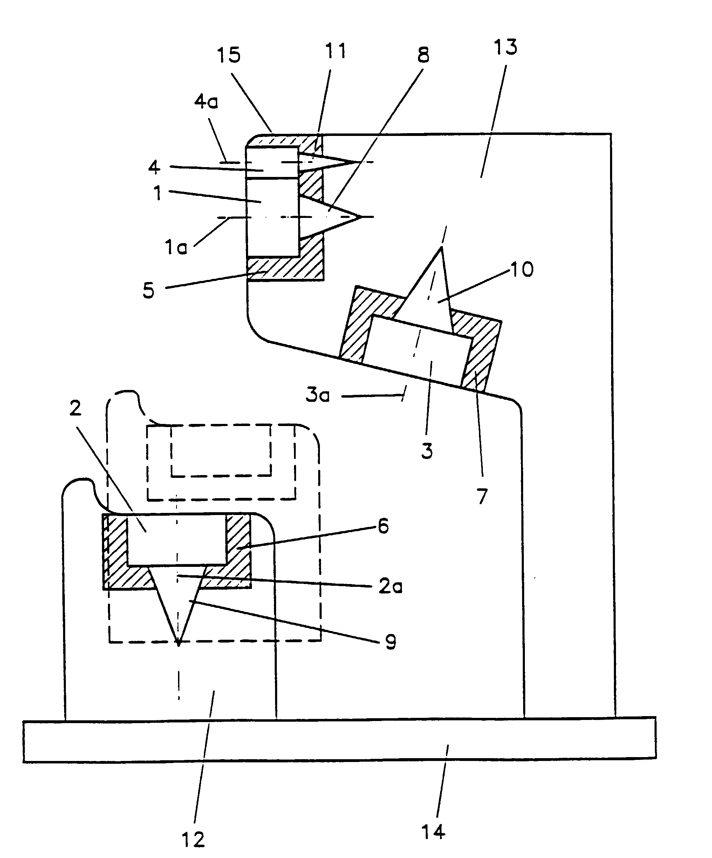

Die Erfindung wird nun an Hand eines Ausführungsbeispiels und der Figur näher erläutert.

Die Figur zeigt den schematischen Aufbau eines Detektorsystems mit vier Detektoren.

Die Detektoren 1, 3 und 4 sind in einem Gehäuse 13 gehaltert, welches außerdem die Abschirmungen 5 und 7, sowie die Signalauswertmodule 8, 10 und 11 trägt. Diese Auswertemodule bestehen aus Photomultiplieren mit Hochspannungsversorgung, Vorverstärker und Hauptverstärker/Diskriminator. Das Gehäuse ist fest mit einer Bodenplatte 14 verbunden, welche auch das Gehäuse 12 für den Detektor 2 mit Abschirmung 6 und Signalauswertemodul 9 trägt.

Die Abschirmungen 5, 6 und 7 bestehen aus 5 cm dickem Blei (auf besonders niedrige Eigenaktivität braucht nicht geachtet zu werden).

Detektor 1 ist auf Lunge, Bronchien und die assoziierten Lymphknoten ausgerichtet. Die Meßanordnung wurde so optimiert, daß der Lungenschwerpunkt bei Personen mit Körpergrößen zwischen 150 cm und 200 cm höchstens 2 cm von der Mittelachse des Detektors entfernt ist. Die Größe und die Abschirmung des Detektors wurden so optimiert, daß bei Personen mit Körpergrößen zwischen 150 cm und 200 cm das Detektorfenster voll abgedeckt wird und bei einer Person mit einer Körpergröße von 170 cm (Referenzmensch) die Detektorempfindlichkeiten für Nukliddepositionen im Bereich der Lunge, des oberen Bauchraums und des unteren Bauchraums im Verhältnis 12:6:1 stehen.

Detektor 2 ist auf den Verdauungstrakt ausgerichtet. Die Meßanordnung wurde so optimiert, daß die Rumpfachse der Person senkrecht auf dem Detektorfenster steht und höchstens 2 cm von der Mittelachse des Detektors entfernt ist. Die Größe des Detektors wurde so optimiert, daß (a) bei Personen mit Körpergrößen zwischen 150 cm und 200 cm das Detektorfenster voll abgedeckt wird und (b) bei einer Person mit einer Körpergröße von 170 cm (Referenzmensch) die Detektorempfindlichkeit für Nukliddepositionen im Bereich der Lunge, des oberen Bauchraums und des unteren Bauchraums im Verhältnis 1:3:20 stehen.

Das kleine Gehäuse 12 ist gegenüber dem Gehäuse 13 verfahrbar. Das Gehäuse 12 dient als Sitzvorrichtung. Die Verfahrwege der Sitzvorrichtung betragen 15 cm in horizontaler Richtung und 15 cm in vertikaler Richtung. Die Anpassung der Sitzvorrichtung erfolgt individuell aufgrund der auf der Identifizierungskarte angegebenen biometrischen Parameter (Körpergröße, Körpergewicht, Brustumfang). Durch die individuelle Anpassung der Sitzposition werden die o.a. genannten Optimierungskriterien gewährleistet.

Detektor 3 ist auf die Oberschenkel und das Knie ausgerichtet. Die Meßanordnung wurde so optimiert, daß bei Personen mit Körpergrößen zwischen 150 cm und 200 cm der Schwerpunkt der Oberschenkel höchstens 3 cm von einer Isoresponse-Kurve (Kurve von Raumpunkten gleicher Empfindlichkeit) des Detektors abweicht und der Mittelpunkt zwischen den Kniekehlen höchstens 5 cm von einem festen Bezugspunkt entfernt ist. Dieser Bezugspunkt liegt in der Mittelachse des Detektors 20 cm vor dem Detektorfenster. Dadurch wird bei allen Personen nahezu die gleiche Empfindlichkeit für homogene Ganzkörperdepositionen erzielt.

Außerdem wurde die Meßanordnung so optimiert, daß die Strahlung aus den dosisrelevanten Organen unter einem Winkel von mindestens 120° einfällt und somit nicht nennenswert zum Meßwert von Detektor 3 beiträgt.

Der Detektor 4 ist bei diesem Beispiel in die Abschirmung 5 integriert. Die Oberseite der Abschirmung 5 kann eine Auflage 15 zur Fixierung des Kinns des Probanden tragen.

Dieser Detektor 4 ist nur bei der Inkorporation von schilddrüsenspezifischen Nukliden wie I-131 und Tc-99m von Bedeutung. Er befindet sich in Höhe der Schilddrüse und hat eine Größe von 6 x 6 x 10 cm. Zum Nachweis der übrigen Nuklide genügt das aus den Detektoren 1 bis 3 gebildete System.

Bei den Detektoren 1 bis 3 handelt es sich um teilabgeschirmte Plastik-Szintillatoren mit den Kristalldimensionen 20 cm x 20 cm x 10 cm. Die Detektoren werden mit einer einfachen Elektronik, bestehend aus Hochspannungsversorgung, Vorverstärker und Hauptverstärker/Diskriminator, betrieben. Die Detektorsignale werden über eine Datenaquisitionseinheit in einen PC eingelesen und dort zur Berechnung der effektiven Äquivalentdosisleistung weiterverarbeitet.

Der Proband setzt sich zur Messung auf die Sitzvorrichtung mit Detektor 2 und löst mit beiden Händen über zwei (nicht dargestellte) Druckknöpfe an den Seiten des Systems die Messung aus. Durch die Anordnung des Stuhls und der Auslöseknöpfe wird eine relativ gut reproduzierbare Meßposition gewährleistet, in der insbesondere der Atemtrakt und der Verdauungstrakt weitgehend unabhängig von der Körpergröße in definiertem Abstand zu den jeweiligen Detektoren angeordnet sind.

Tabelle 2:

| Depot S | Empfindlichkeit | $e_i(S)$ | (Imp/s/Bq Co-60) |
|---|---|---|---|
| | Det. 1 | Det. 2 | Det. 3 |
| Ganzkörper | 0,022 | 0,026 | 0,016 |
| Lunge | 0,114 | <0,001 | <0,001 |
| Leber | 0,058 | 0,014 | <0,001 |
| Dünndarm | 0,010 | 0,067 | <0,001 |
| Oberer Dickdarm | 0,014 | 0,058 | <0,001 |
| Unterer Dickdarm | <0,001 | 0,110 | 0,001 |

Die Tabelle 2 zeigt als Beispiel die für diese Meßposition ermittelten Empfindlichkeiten der Detektoren 1 bis 3 für homogene Co-60 Depositionen im Ganzkörper sowie in verschiedenen dosisrelevanten Organen. Multipliziert man diese Werte mit den Wichtungsfaktoren

$$\alpha_i = 0,91$$

$$\alpha_2 = 2,72 \qquad\qquad (12)$$

$$\alpha_3 = -0,263$$

so ergeben sich nach Gl. (7) die in Tabelle 3 aufgeführten Werte der Systemempfindlichkeit m(S).

Tabelle 3:

| Deopt S | Systemempfindlichkeit m(S) (imp/s/Bq Co-60) |
|---|---|
| Ganzkörper | 0,049 |
| Lunge | 0,104 |
| Leber | 0,091 |
| Dünndarm | 0,191 |
| Oberer Dickdarm | 0,171 |
| Unterer Dickdarm | 0,297 |

Vergleicht man diese Werte mit den in Tab. 1 aufgeführten Dosisleistungsfaktoren h(S), so zeigt sich, daß die durch Gl. (10) gegebene Beziehung für alle Depositionen von Co-60 in guter Näherung erfüllt ist. Für den Kalibrierfaktor K ergibt sich nach Gl. (10):

$$K = 4,1 +/- 0,3 \text{ nSv/d pro imp/s} \qquad\qquad (13)$$

Demnach kann man mit dem hier konzipierten Detektorsystem bei allen Verteilungsmustern von Co-60 die effektive Äquivalentdosisleistung prinzipiell mit einem Fehler von weniger als 10 % direkt ermitteln.

Bei anderen Nukliden bzw. Nuklidgemischen kann man nach entsprechender Anpassung der Wichtungsfaktoren $\alpha_i$ und des Kalibrierfaktors K eine vergleichbare Genauigkeit erzielen. Wenn die involvierten Nuklide bzw. die Nuklidkomposition nicht bekannt ist, muß man mit Standardwerten arbeiten und damit einen größeren Fehler in Kauf nehmen. Aufgrund der vorliegenden Ergebnisse ist es zweckmäßig, als Standardwerte die durch (12) und (13) gegebenen Werte für Co-60 zu benutzen. Mit diesen Standardwerten ergeben sich die in Tab. 4 angegebenen Werte für die Systemempfindlichkeit m(S) für homogene Ganzkörperdepositionen einiger ausgewählter Nuklide. Bei diesen Nukliden handelt es sich um eine Auswahl der wichtigsten im kerntechnischen Arbeitsbereich auftretenden Spalt- und Aktivierungsprodukte.

Tabelle 4:

| Nuklid | Systemempfindlichkeit m(S) (Imp/s/Bq) |
|---|---|
| Co-60 | 0,049 |
| Cs-137 | 0,025 |
| Zn-65 | 0,014 |
| Sb-124 | 0,048 |
| Co-58 | 0,037 |
| Ag-110m | 0,088 |
| Mn-54 | 0,028 |
| Zr-95 | 0,057 |
| Ce-144 | 0,0026 |
| Ba-140 | 0,068 |
| Ru-106 | 0,010 |

Tabelle 5:

| Nuklid | Relative Dosisleistungsanzeige für homogene Depositionen in | | | | | | |
|---|---|---|---|---|---|---|---|
| | Lunge | SI | ULI | LLI | Leber | sonstige | GK[a] |
| Co-60 | 0,99 | 1,09 | 0,88 | 1,01 | 1,07 | | 1,00 |
| Cs-137 | 0,44 | | | | | | 1,01 |
| Zn-65 | 1,42 | 1,18 | 1,11 | 1,39 | | 1,55[b] | 1,31 |
| Sb-124 | 0,50 | 0,84 | 0,55 | 0,64 | 1,04 | 0,93[b] | 0,93 |
| Co-58 | 2,00 | 1,78 | 1,42 | 1,83 | 2,16 | | 1,99 |
| Ag-110m | 1,67 | 1,41 | 1,26 | 2,19 | 1,56 | 2,16[c] | 1,72 |
| Mn-54 | 2,02 | 1,59 | 1,42 | 1,92 | 1,63 | | 1,84 |
| Zr-95 | 1,23 | 1,51 | 1,07 | 1,30 | | 0,86[b] | 2,11 |
| Ce-144 | 0,01 | | 0,02 | 0,02 | 0,03 | 0,02[b] | 0,09 |
| Ba-140 | 0,36 | 0,70 | 0,42 | 0,48 | | 1,07[c] | 0,90 |
| Ru-106 | 0,04 | 0,11 | 0,05 | 0,06 | | 0,03[c] | 0,14 |

a) Ganzkörper;

b) Knochen;

c) Mageninhalt

Mit den in Tab. 4 aufgeführten Systemempfindlichkeiten ergeben sich die in Tab. 5 zusammengestellten Werte für die relative Dosisleistungsanzeige des Detektorsystems bei Standardeinstellung. Demnach wird ein homogenes Cs-137-Ganzkörperdepot in der Standardeinstellung nahezu exakt bewertet, während ein Cs-137-Lungendepot um etwa den Faktor 2 unterbewertet ist. Dies ist jedoch radiologisch ohne Bedeutung, da das Lungendepot bei Cs-137-Inkorporationen keinen nennenswerten Beitrag zur Gesamtexposition liefert. Die Nuklide Zn-65, Co-58, Ag-ll0m, Mn-54 und Zr-95 werden in der Standardeinstellung leicht bis mäßig überbewertet, während Sb-124 und Ba-140 leicht bis mäßig unterbewertet werden. Die Nuklide Ce-144 und Ru-106 werden vergleichsweise stark unterbewertet. Wenn diese beiden Nuklide in nennenswertem Umfang zur Gesamtdosis beitragen, kann folglich nicht mit der Standareinstellung gearbeitet werden. Ansonsten können mit der Standardeinstellung recht gute Ergebnisse erzielt werden. Mittelt man die für die angegebenen Nuklide mit Ausnahme von Ru-106 und Ce-144 berechneten Werte der relativen Dosisanzeige (RD), so erhält man:

| | |
|---|---|
| Ganzkörperdepot: | RD = 1,42 +/- 0,49 |
| Lungendepot: | RD = 1,18 +/- 0,65 |
| SI-Depot: | RD = 1,26 +/- 0,38 |
| ULI-Depot: | RD = 1,01 +/- 0,38 |
| LLI-Depot: | RD = 1,34 +/- 0,61 |
| Leberdepot: | RD = 1,49 +/- 0,46 |
| Knochendepot: | RD = 1,11 +/- 0,38 |

Demnach werden Ganzkörperdepots im Mittel um 42 % überbewertet, wobei mit einer mittleren Streubreite in Höhe von etwa 35 % zu rechnen ist. Lungendepots werden im Mittel um 18 % überbewertet, allerdings ist hier mit einer mittleren Streubreite in Höhe von etwa 55 % zu rechnen. Bildet man das quadratische Mittel der für die verschiedenen Depositionen berechneten relativen Streubreiten, so erhält man einen mittleren Fehler in Höhe von etwa 38 %. Das mittlere Überbewertungspotential liegt bei etwa 26 %.

Zusammenfassend kann man folgendes feststellen:

1. Bei Inkorporationen von Co-60 kann die effektive Äquivalentdosisleistung in der Standardeinstellung prinzipiell mit einem Fehler von weniger als 10% gemessen werden.

2. Bei Inkorporationen von anderen Spalt- und Aktivierungsprodukten mit hinreichend intensiver Gamma-Strahlungskomponente wie z.B. Cs-137, Zn-65, Sb-124, Co-58, Ag-IlOm, Mn-54, Zr-95 und Ba-140 wird die effektive Äquivalentdosisleistung in der Standardeinstellung im Mittel um etwa 26 % überbewertet, wobei mit einem mittleren Fehler von etwa 40 % gerechnet werden muß.

3. Bei Inkorporationen von Spalt- und Aktivierungsprodukten mit schwacher Gamma-Strahlungskomponente wie z.B. Ru-106 und Ce-144 müssen die Wichtungs- und Kalibrierfaktoren entsprechend der Nuklidkomposition geändert werden. Bei guter Anpassung der Einstellwerte kann die effektive Äquivalentdosisleistung bei diesen Nukliden prinzipiell mit einem Fehler von weniger als 20 % gemessen werden.

Die untere Nachweisgrenze wird im wesentlichen von der Genauigkeit der Nulleffektvorhersage bei den verschiedenen Detektoren bestimmt. Der Nulleffekt wird zum einen durch die Intensität und die Haupteinfallsrichtung der Umgebungsstrahlung und zum anderen durch die Abschirmwirkung der Probanden bestimmt. Die erstgenannte Komponente kann sehr genau durch regelmäßige Nulleffektmessungen kontrolliert werden, so daß sich der diesbezügliche Fehler auf den zählstatistischen Fehler reduziert. Bei der zweitgenannten Komponente müssen die individuellen Körperproportionen berücksichtigt werden.

Aufgrund der von uns durchgeführten Testmessungen beträgt der Nulleffekt des in Brusthöhe angeordneten Detektors bei einem Probanden mit einem Körpergewicht von etwa 70 kg und durchschnittlichen Körperproportionen etwa 12000 imp/min. Bei der für die Routineüberwachung angestrebten Meßzeit von 20 s entspricht dies einer Nulleffektzählrate von etwa 4000 imp bzw. einem mittleren zählstatistischen Fehler von etwa 60 imp. Bedingt durch die unterschiedliche Abschirmwirkung der Probanden ist die individuelle Schwankungsbreite der Nulleffektzählrate jedoch deutlich größer als 60 imp pro 20 s. Die Messungen zeigten allerdings, daß die Abschirmwirkung außerordentlich gut mit dem Brustumfang der Probanden korreliert. So kann bei Personen mit einem Brustumfang zwischen 80 und 120 cm die Nulleffektzählrate des Detektors mit Hilfe einer empirischen Formel mit einer mittleren Abweichung von etwa 80 imp pro 20 s abgeschätzt werden. Dieser Wert liegt nur knapp über dem bei dieser Meßzeit zu erwartenden zählstatistischen Fehler. Auch das Gewicht der Probanden kann als Steuerparameter herangezogen werden, wobei sowohl bei dem in Brusthöhe angeordnetem Detektor als auch bei dem unter der Sitzfläche angeordnetem Detektor mit einem mittleren Fehler in Höhe von etwa 120 imp pro 20 s zu rechnen ist. bei dem über den Oberschenkeln angeordnetem Detektor ist die durch den Körper bedingte Abschirmwirkung relativ gering, so daß keine individuellen Korrekturen erforderlich sind. Bei diesem Detektor ist mit einem mittleren Fehler in Höhe von etwa 80 imp pro 20 s zu rechnen.

Die genannten Fehlerbreiten beziehen sich auf den Fall, daß das Detektorsystem in einem Raum ohne spezielle Abschirmeinrichtungen installiert ist. Insofern stellen die genannten Fehlerbreiten eine obere Grenze der in der Praxis erzielbaren Werte dar.

Für die weiteren Betrachtungen wird aus Konservativitätsgründen angenommen, daß der mittlere Fehler in der Nulleffektvorhersage bei allen drei Detektoren bei 120 imp pro 20 s liegt. Folglich liegt der mittlere Fehler der Nettozählrate bei jedem Detektor bei etwa 140 imp pro 20 s.

Nach Gln. (9,11) gilt für die effektive Äquivalentdosisleistung

$$H_{eff}' = K \cdot M = K \cdot \Sigma_i[\alpha_i \cdot Z_i] \qquad (14)$$

Dabei ist $Z_i$ die durch die Inkorporation bedingte Impulsrate des i-ten Detektors. Aus Gl. (14) ergibt sich für den mittleren Fehler der effektiven Äquivalentdosisleistung

$$S^2(H_{eff}') = S^2(K) \cdot (\Sigma_i[\alpha_i \cdot Z_i])^2 + K \cdot \Sigma_i[(\alpha_i)^2 \cdot S^2(Z_i)] \qquad (15)$$

Hierbei ist

$s^2(H_{eff}')$      die mittlere quadratische Schwankungsbreite von $H_{eff}'$
$s^2(K)$      die mittlere quadratische Schwankungsbreite von K
$S^2(Z_i)$      die mittlere quadratische Schwankungsbreite von $Z_i$

Bei Standardeinstellung des Detektorsystems gilt im Fall von Co-60-Inkorporation nach Gl. (13):

$$K = 4,1 +/- 0,3 \text{ nSv/d pro imp/s}$$

Im Fall von anderen Nukliden mit hinreichend hoher Gamma-Strahlungskomponente kann für K aufgrund der o. a. Betrachtungen angenommen werden:

$$K = 3,3 +/- 1,3 \text{ nSv/d pro imp/s}$$

Die untere Nachweisgrenze wird hier zweckmäßigerweise durch die Forderung definiert, daß der mittlere Fehler der effektiven Äquivalentdosisleistung nicht größer als 50 % sein darf. Mit dieser Forderung ergeben sich bei Zugrundelegung von $s(Z_i)$ = 140 imp pro 20 s entsprechend $s(Z_i)$ = 7 imp/s untere Nachweisgrenzen in Höhe von

$$H_{eff}'\phi = 0,17 \ \mu\text{Sv/d entsprechend } 62 \ \mu\text{Sv/a}$$

bei Inkorporation von Co-60 bzw. in Höhe von

$$H_{eff}'_O = 0,27 \ \mu\text{Sv/d entsprechend } 99 \ \mu\text{Sv/a}$$

im Fall von Inkorporationen unbekannter Gemische von Nukliden mit hinreichend hoher Gamma-Strahlungskomponente.

Diese Nachweisgrenzen beziehen sich auf eine einmalige Routinemessung von 20 s Dauer. Bei regelmäßigen Routinemessungen in wöchentlichem Abstand kann man davon ausgehen, daß die auf das Jahr bezogene Nachweisgrenze sicher kleiner als die angegebenen Werte sind. Folglich kann man zusammenfassend feststellen, daß die untere Nachweisgrenze des Verfahrens bei Inkorporationen von Nukliden mit hinreichend hoher Gamma-Strahlungskomponente kleiner als 0,1 mSv/a ist. Sie ist damit vergleichbar mit der unteren Nachweisgrenze der in der externen Dosimetrie angewandten Verfahren.

**Patentansprüche**

1. Detektorsystem zur direkten internen Dosimetrie an einer Person, mit mindestens drei Detektoren zum Nachweis von Gamma-Quanten und Mitteln zur Detektorhalterung bei dem ein erster Detektor (1), dessen Mittelachse (la) auf den Lungenschwerpunkt der Person weist, ein zweiter Detektor (2), dessen Mittelachse (2a) auf den Schwerpunkt des Verdauungstraktes der Person weist, und ein dritter Detektor (3), dessen Mittelachse (3a) auf den Beinbereich der Person weist, vorgesehen sind, und bei dem die Detektoren (1, 2, 3) von zum Körper der Person hin offenen Bleiabschirmungen (6, 7, 8) umgeben sind, dadurch gekennzeichnet, daß der zweite Detektor (2) in einem hockerähnlichen Gehäuse (12) derart gehaltert ist, daß die nicht abgeschirmte Fläche des Detektors (2) nach oben weist.

2. Detektorsystem nach Anspruch 1, dadurch gekennzeichnet, daß die nicht abgeschirmte Fläche des ersten Detektors (1) der Brust der Person in aufrechter Sitzposition gegenüberliegt, wobei die Mittelachse (2a) horizontal verläuft.

3. Detektorsystem nach Anspruch 1 oder 2, dadurch gekennzeichnet, daß die nicht abgeschirmte Fläche des dritten Detektors (3) den Unterschenkeln der Person in aufrechter Sitzposition gegenüberliegt, wobei der Winkel zwischen der Mittelachse (3a) und der Vertikalen einen Wert zwischen 0° und 30° aufweist.

4. Detektorsystem nach einem der Ansprüche 1 bis 3, gekennzeichnet durch einen weiteren Detektor (4), dessen Mittelachse (4a) auf die Schilddrüse der Person weist.

5. Detektorsystem nach einem der Ansprüche 1 bis 4, dadurch gekennzeichnet, daß das Gehäuse (12) horizontal und vertikal verschiebbar ausgebildet ist.


## Claims

1. Detector system for the direct internal dosimetry in a person, having at least three detectors for detecting gamma quanta and means for holding the detectors, wherein a first detector (1) is provided, the central axis (la) of which points towards the centre of mass of the lungs of the person, a second detector (2) is provided, the central axis (2a) of which points to the centre of mass of the digestive tract of the person, and a third detector (3) is provided, the central axis (3a) of which points towards the leg area of the person, and wherein the detectors (1, 2, 3) are surrounded by protective lead screens (6, 7, 8), which are open towards the body of the person, characterised in that the second detector (2) is mounted in a stool-like housing (12) in such a manner that the non-screened area of the detector (2) points upwardly.

2. Detector system according to claim 1, characterised in that the non-screened area of the first detector (1) lies opposite the chest of the person when sitting in an upright position, the central axis (2a) extending horizontally.

3. Detector system according to claim 1 or 2, characterised in that the non-screened area of the third detector (3) lies opposite the tibias of the person when sitting in an upright position, the angle between the central axis (3a) and the vertical having a value of between 0° and 30°.

4. Detector system according to one of claims 1 to 3, characterised by an additional detector (4), the central axis (4a) of which points towards the thyroid gland of the person.

5. Detector system according to one of claims 1 to 4, characterised in that the housing (12) is configured to be horizontally and vertically displaceable.


## Revendications

1. Système détecteur pour la dosimètrie interne directe sur une personne, ayant au moins trois détecteurs pour détecter des quanta gamma et des moyens pour la fixation des détecteurs , système dans lequel sont prévus un premier détecteur (1), dont l'axe central (1a) indique le centre de gravité des poumons de la personne, un deuxième détecteur (2) dont l'axe central (2a) indique le centre de gravité du tube digestif de la personne, et un troisième détecteur (3), dont l'axe central (3a) indique la zone de la jambe de la personne et dans lequel les détecteurs (1, 2, 3) sont entamés d'écrans de plomb (6, 7, 8) ouverts en direction du corps de la personne,
caractérisé en ce que
le deuxième détecteur (2) est maintenu dans un boîtier (12) semblable à un tabouret, de façon que la surface non protégée du détecteur regarde vers le haut.

2. Système détecteur selon la revendication 1,
caractérisé en ce que
la surface non protégée du premier détecteur (1) est située en face de la poitrine de la personne en position d'ajustage vertical, l'axe central (2a) se présentant horizontal.

3. Système détecteur selon la revendication 1 ou 2,
caractérisé en ce que
la surface non protégée du troisième détecteur (3) est située en face des jambes de la personne en position d'ajustage vertical, l'angle entre l'axe central (3a) et la verticale comportant une valeur comprise entre 0° et 30°.

4. Système détecteur selon une des revendications 1 à 3,
caractérisé en ce que
un autre détecteur (4), dont l'axe central (4a) regarde la glande thyroïde de la personne.

5. Système détecteur selon une des revendications 1 à 4,
caractérisé en ce que
le boîtier (12) est déplaçable horizontalement et verticalement.